# EUROPEAN PATENT SPECIFICATION

(11) **EP 1 802 262 B1**
(45) Date of publication and mention of the grant of the patent: **21.01.2015**
(21) Application number: 05816155.5
(22) Date of filing: 17.10.2005
(51) Int. Cl.: A61F 13/62

(54) **BONDING PATTERNS FOR CONSTRUCTION OF A KNITTED FABRIC LANDING ZONE**
VERBINDUNGSMUSTER ZUR HERSTELLUNG EINES EINLAGEBEREICHS AUS MASCHENWARE
MOTIFS DE LIAISON POUR CONSTRUCTION DE ZONE DE RECEPTION EN TISSU TRICOTE

(30) Priority: 18.10.2004 US 967730
(43) Date of publication of application: 04.07.2007
(60) Divisional application: 08020423.3
(73) Proprietor: The Procter & Gamble Company, Cincinnati, OH 45202 (US)
(72) Inventor: HORN, Thomas, Alexander, 65719 Hofheim (DE); KLINE, Mark, James, Okeana, Ohio 45053 (US); BALDAUF, Georg, 48366 Laer (DE)
(74) Representative: Heide, Ute
(86) International application number: PCT/US2005/038278
(87) International publication number: WO 2006/045118

(56) References cited:
- EP-A- 0 583 081
- EP-A1- 1 579 779
- US-A- 5 699 593

## Description

### FIELD OF THE INVENTION

The present invention relates to bonding patterns for construction of knitted fabric fastener female portions. More particularly, bonding patterns for construction of knitted fabric landing zones for a disposable absorbent article.

### BACKGROUND

Refastenable mechanical fastening systems are used in a wide number of consumer product applications. Such fastening systems are used to connect one portion of an article to another portion of the same article, or another article, or device. Typically, refastenable mechanical fastening systems comprise a receiving (or female) member and an engaging (or male) member. The receiving member generally comprises a plurality of fibrous engaging elements (e.g., loops). The engaging member generally comprises a plurality of hook elements. The hooks are capable of entangling with the loops to form a connection between the engaging and receiving members. Prior art fastening systems are disclosed in EP 0583081, US 5699593 and EP 1579779.

One particular type of receiving member is a knitted fabric landing zone. Knit fabrics are generally made from three yarn components: chains, wefts and loops. The chains generally run parallel to the knitted fabric's longitudinal centerline (which typically corresponds with the longitudinal centerline of the absorbent article). The wefts generally run across and between the chains in a path which is substantially parallel to the knitted fabric's lateral centerline (which typically corresponds with the lateral centerline of the absorbent article). The chains and wefts may be weaved and/or knitted together to form the base structure of the knitted fabric. The loops may be weaved and/or knitted to extend from a first side of said base structure. The outwardly-extending loops provide the engagement functionality for the hooks of the engaging member. An underlying substrate may be joined to a second side of said base structure in order to provide structural support and integrity for the base structure and adjoined loops. The underlying substrate may be directly or indirectly joined to the absorbent article.

When using a knitted fabric landing zone in a disposable absorbent article, the following considerations should be contemplated: (1) connection performance [defined herein as the ability of the hook and loop to remain engaged under certain expected peel forces and/or shear forces], (2) structural integrity [defined herein as the ability of the fiber base structure to remain engaged with the underlying substrate] and (3) cost.

When considering connection performance and structural integrity, three common test methods are used within the industry: (a) Dynamic Shear Test [In practice, this test method is often a measure of the hook-to-loop strength or engagement which is one mode of failure. Even further, this test methods measures the resistance to disengagement in response to loads generally in the X-direction. Given the high basis weights of the loop material that are commonly used, shear is typically less of a concern than peel; however, when lower basis weights are used, shear becomes more of a concern.], (b) Peel Test [In practice, an average peak value of 5 newtons is often desirable to ensure that the fastening system sufficiently fastens and a maximum peak value of 12 newtons is sometimes desirable to ensure that the fastening system is not too difficult to be unfastened by the caregiver. This test method measures the resistance to disengagement in response to loads generally in the Z-direction] and (c) CD (cross-directional) Bond Strength of knitted fabric to substrate [In practice, an average value of at least about 6 newtons/25.4 mm is sometimes desirable to ensure that the fabric base structure is sufficiently bonded to the underlying substrate. This test method measures the resistance to fabric-to-substrate delamination/separation in response to loads generally in the Z-direction]. When designing a suitable refastenable mechanical fastening system, particularly one having a knitted fabric landing zone, the competing interests of the Peel Test and CD Bond Strength Test make it difficult to construct the optimal fastening system. More specifically, the addition of more adhesive between the fabric base structure and the underlying substrate should increase the CD Bond Strength value; however, said adhesive will frequently migrate through said base structure and to the loops on the opposing side, and in doing so, said loops will no longer be free for subsequent fastening to the hooks. Attempts in the prior art have been made to address these competing interests.

One such attempt in the prior art includes a process for targeting adhesive application only onto the chains within the fabric base structure. While this process is designed to provide adhesive in the areas in which it's needed (i.e., chains) and not in the areas in which it's undesirable (i.e., loops), this process typically involves the risk of potentially gluing down portions of the loops within the area of the chains where most of the adhesive is present, especially if mis-tracking of the adhesive occurs. Additionally, such an approach involves the added process complexity and costs associated with a targeted but random (due to variations) glue deposition on the fabric base structure (i.e., on the chains of the knitted fabric).

Another such attempt is to modify the fabric geometric knit patterns in a way that the fabric functional loops are better protected in the lamination step to reduce the likelihood to glue down the functional loops - thus better preserving the loop functionality in the glued areas. However, this approach requires the use of specially designed fabric patterns and thus reduces the degrees of freedom in fabric selection.

Another such attempt in the prior art includes a process for targeting adhesive application only on the underlying substrate. When the adhesive is transferred from the transfer roll to the substrate, a smooth backing roll is used to create a transfer nip. The use of such smooth backing roll results in a 100% coverage of adhesive to the substrate. After applying the adhesive to the underlying substrate, the knitted fabric is joined to the substrate. In so doing, however, an undesirable amount of adhesive will still migrate through the base structure and to the loops on the opposing side.

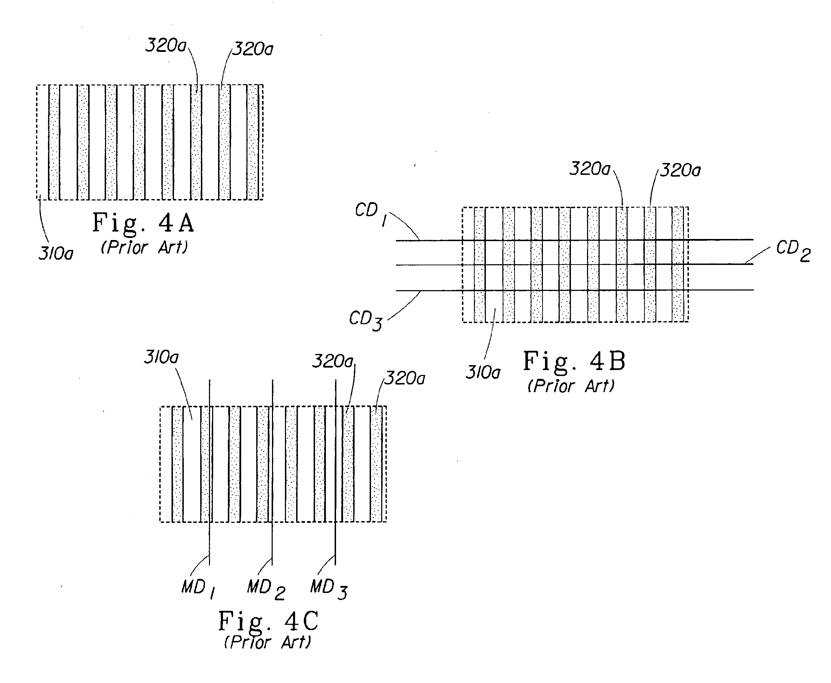

In further examining the prior art, it is recognized that a wide variety of adhesive application patterns are known. As mentioned above, for example, 100% coverage of adhesive to the underlying substrate is sometimes used. Such a 100% coverage pattern should result in a high CD Bond Test value; however, said application pattern should also result in a low Peel Test value because many of the loops have been contaminated with adhesive. Another prior art pattern includes partial adhesive coverage in a striped configuration (see FIGS. 4a-7a). Such a striped pattern should result in a medium value for both the CD Bond Test and the Peel Test. More specifically, as the spacing between the stripes increases, the CD Bond Test value should decrease while the Peel Test value should increase, and vice versa. Yet another prior art pattern includes random adhesive application (e.g., spiral applicators) having less than 100% coverage of adhesive. Such a random pattern should result in a medium value for both the CD Bond Test and the Peel Test; however, the inherent randomness of the pattern sometimes results in a variable connection performance. Yet another prior art pattern involves a bonding pattern having CD and MD bond lines, however, such prior art is directed to the bonding of a nonwoven to an underlying substrate which does not involve nor address the unique issues of knitted fabric (e.g., it is desirable to place adhesive on the chains but not on the loops of the knitted fabric and to prevent the presence of frayed edges).

Another necessary consideration of structural integrity, as well as overall aesthetic appearance, is the percent coverage of adhesive along the perimeter of the knitted fabric landing zone structure. More specifically, it is desirable to have the entire perimeter of the knitted fabric adhered to the underlying substrate in order to minimize the appearance and negative impact of frayed edges; however, in doing so, the field of the knitted fabric should minimally be adhered to the underlying substrate in order to maximize the Peel Test value. When applying this additional consideration to the prior art examples discussed above, it is further appreciated that the task of optimizing the adhesive application pattern is even more difficult. For example, a 100% coverage pattern does in fact provide the most optimal perimeter coverage; however, said pattern may have a low Peel Test value. In another example, the striped pattern will have gaps along the perimeter which correspond to the spacing of the stripes; such that, as the spacing is increased to improve the Peel Test value, the gaps along the perimeter will negatively increase. In yet another example, the random pattern inherently will not provide predictable gap sizes and locations along the perimeter. Consequently, none of the cited prior art examples properly address this perimeter adhesion consideration.

To only further complicate the already difficult task of optimizing the adhesive print pattern, the way in which the knitted fabric is to be cut during processing must also be considered. More specifically, the knitted fabric web may be cut along its longitudinal axis (i.e., machine direction) in order to reduce a parent roll into smaller rolls, or even just to cut the web to a desired width. Additionally, the knitted fabric web may be cut along its lateral axis (i.e., cross direction) in order to provide patches (e.g., landing zones) having a desired length. Consequently, the adhesive print pattern must be sufficiently robust enough to be cut in both the machine and cross directions without resulting in unduly large gaps along its perimeter; otherwise, frayed edges may appear and/or structural integrity may fail.

Once an adhesive print pattern is technically selected, the equally important consideration of cost must also be satisfied. For example, in the prior art it is known that to overcome the negative effects of adhesive migrating to the loops, one skilled in the art may increase the basis weight of the knitted fabric. A higher basis weight of knitted fabric will (a) provide more loops, which may result in a larger number of loops not being contaminated by said adhesive and (b) provide a more dense fiber layer to further restrict said migration. However, a higher basis weight material is often cost prohibited, especially in the marketplace of absorbent articles.

Given all the considerations above, it is desirable to provide a knitted fabric structure having a novel bonding pattern which satisfactorily meets the requirements of structural integrity, connection performance, process cutting and costs. Additionally, it is desirable to provide a regularly repeating bonding pattern having less than 100 percent coverage area and enabling greater degrees of freedom in balancing the requirements of structural integrity, connection performance, process cutting and costs. Additionally, it is desirable to provide a process which is not limited to specially-designed knitted fabric structures. Additionally, it is desirable to provide a process that allows for the tailoring of bonding patterns based upon the geometry (e.g., chain-to-chain distance, weft design, and loop density, etc.) of the knitted fabric being used.

### SUMMARY OF THE INVENTION

A knitted fabric female fastening portion for a mechanical fastener having a knitted fabric, an underlying substrate, and a bonding layer is provided herein. Said knitted fabric having a first surface and a second surface, said first surface being capable of engaging with a male fastening portion. Said underlying substrate being joined to said second surface of said knitted fabric. Said bonding layer being located between said knitted fabric and underlying substrate, said bonding layer bonding said knitted fabric and underlying substrate together. Said bonding layer having a first plurality of non-intersecting bond lines and a second plurality of non-intersecting bond lines which are combined to form a pattern of intersecting bond lines that define tessellating pattern elements. The pattern of intersecting bond lines has a CD percent coverage standard deviation not equal to 0% and an MD percent coverage standard deviation not equal to 0%. The pattern of intersecting bond lines may have an amplitude greater than 0 mm to about 3 mm. The pattern of intersecting bond lines has a gap from about 2 mm to about 6 mm. The bond lines may be continuous or discontinuous, linear or non-linear, having same or varying thickness. The first plurality of bond lines may have a different thickness than the second plurality of bond lines. The first plurality of bond lines may have a different spacing than the second plurality of bond lines. The bonding layer may be an adhesive.

A knitted fabric female fastening portion for a mechanical fastener having a knitted fabric and an underlying substrate is provided herein. Said knitted fabric having a first surface and a second surface, said first surface being capable of engaging with a male fastening portion. Said underlying substrate being joined to said second surface of said knitted fabric by a fusion bond. Said fusion bonding having a bond pattern. Said bond pattern having a first plurality of non-intersecting bond lines and a second plurality of non-intersecting bond lines which are combined to form a pattern of intersecting bond lines that define tessellating pattern elements. The pattern of intersecting bond lines has a CD percent coverage standard deviation not equal to 0% and an MD percent coverage standard deviation not equal to 0%. The pattern of intersecting bond lines may have an amplitude greater than 0 mm to about 3 mm. The pattern of intersecting bond lines may have a gap from about 2 mm to about 6 mm.

The knitted fabric female fastening portion of the present invention may be used in disposable absorbent articles, body wraps, clothing, packaging, feminine hygiene products, bandages, bibs, food wraps, abrasive systems, cleaning systems and polishing systems.

### BRIEF DESCRIPTION SHOWN IN THE DRAWINGS

While the specification concludes with claims particularly pointing out and distinctly claiming the subject matter that is regarded as the present invention, it is believed that the invention will be more fully understood from the following description taken in conjunction with the accompanying drawings. None of the drawings are necessarily to scale.
FIG. 1 is a schematic perspective view of a conventional diaper;
FIG. 2a is a schematic exploded view of a conventional female fastener portion;
FIG. 2b is a schematic exploded view of a conventional knitted fabric;
FIG. 3a is a schematic top elevational view of a conventional bond pattern having 100% coverage;
FIG. 3b is a schematic top elevational view of the bond pattern in FIG. 3a undergoing CD analysis;
FIG. 3c is a schematic top elevational view of the bond pattern in FIG. 3a undergoing MD analysis;
FIG. 4a is a schematic top elevational view of a conventional bond pattern having striped coverage;
FIG. 4b is a schematic top elevational view of the bond pattern in FIG. 4a undergoing CD analysis;
FIG. 4c is a schematic top elevational view of the bond pattern in FIG. 4a undergoing MD analysis;
FIG. 5a is a schematic top elevational view of a conventional bond pattern having striped coverage;
FIG. 5b is a schematic top elevational view of the bond pattern in FIG. 5a undergoing CD analysis;
FIG. 5c is a schematic top elevational view of the bond pattern in FIG. 5a undergoing MD analysis;
FIG. 6a is a schematic top elevational view of a conventional bond pattern having striped coverage;
FIG. 6b is a schematic top elevational view of the bond pattern in FIG. 6a undergoing CD analysis;
FIG. 6c is a schematic top elevational view of the bond pattern in FIG. 6a undergoing MD analysis;
FIG. 7a is a schematic top elevational view of a conventional bond pattern having striped coverage;
FIG. 7b is a schematic top elevational view of the bond pattern in FIG. 7a undergoing CD analysis;
FIG. 7c is a schematic top elevational view of the bond pattern in FIG. 7a undergoing MD analysis;
FIG. 8 shows a summary chart of the prior art bond patterns discussed herein;
FIG. 9a shows an exemplary bond pattern in accordance with the present invention;
FIG. 9b is a schematic top elevational view of the bond pattern in FIG. 9a undergoing CD analysis;
FIG. 9c is a schematic top elevational view of the bond pattern in FIG. 9a undergoing MD analysis;
FIG. 10a shows an exemplary bond pattern in accordance with the present invention;
FIG. 10b is a schematic top elevational view of the bond pattern in FIG. 10a undergoing CD analysis;
FIG. 10c is a schematic top elevational view of the bond pattern in FIG. 10a undergoing MD analysis;
FIG. 11a shows an exemplary bond pattern in accordance with the present invention;
FIG. 11b is a schematic top elevational view of the bond pattern in FIG. 11a undergoing CD analysis;
FIG. 11c is a schematic top elevational view of the bond pattern in FIG. 11a undergoing MD analysis;
FIG. 12a shows an exemplary bond pattern in accordance with the present invention;
FIG. 12b is a schematic top elevational view of the bond pattern in FIG. 12a undergoing CD analysis;
FIG. 12c is a schematic top elevational view of the bond pattern in FIG. 12a undergoing MD analysis;
FIG. 13 shows a summary chart of the bond patterns in accordance with the present invention;
FIG. 14a shows an exemplary portion of an adhesive print pattern in accordance with the present invention;
FIG. 14b shows a tessellating pattern element with nonlinear side edges;
FIG. 14c shows a tessellating pattern element with nonlinear side edges having an amplitude which is larger than the amplitude in FIG. 14b;
FIG. 15 shows a chart of exemplary print patterns having varying percent coverage areas and varying amplitudes;
FIG. 16a shows an exemplary knitted fabric and an underlying print pattern which are known in the prior art;
FIG. 16b shows adhesive and loops of FIG. 16a being misaligned;
FIG. 17a shows an exemplary knitted fabric and an underlying print pattern having wide lines of adhesive which are known in the prior art;
FIG. 17b shows adhesive and loops of FIG. 17a being misaligned;
FIG. 18a shows an exemplary knitted fabric and an underlying print pattern in accordance with the present invention;
FIG. 18b shows adhesive and loops of FIG. 18a being misaligned;
FIG. 19a shows an exemplary knitted fabric and an underlying print pattern having wide lines of adhesive in accordance with the present invention;
FIG. 19b shows adhesive and loops of FIG. 19a being misaligned;
FIG. 20a shows a novel print pattern having broken lines;
FIG. 20b shows a novel print pattern having discrete points or dots;
FIG. 20c shows a novel print pattern having non-linear lines;
FIG. 20d shows a novel print pattern having non-linear, broken lines;
FIG. 21 shows a chart which further demonstrates the novel aspects of the present invention;
FIG. 22 shows an alternative embodiment of an absorbent article wherein the landing zone substantially covers the entire surface of said absorbent article; and
FIG. 23 depicts an exemplary embodiment of the present invention wherein female portion may comprise an underlying substrate layer and a knitted fabric layer which are fusion bonded together using bonding pattern rather than using a bonding agent (e.g., adhesive).

### DETAILED DESCRIPTION OF THE INVENTION

### Definitions:

The term "absorbent article" herein refers to devices which absorb and contain body exudates and, more specifically, refers to devices which are placed against or in proximity to the body of the wearer to absorb and contain the various exudates discharged from the body, such as: incontinence briefs, incontinence undergarments, absorbent inserts, diaper holders and liners, feminine hygiene garments and the like.

The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner).

The term "diaper" herein refers to an absorbent article generally worn by infants and incontinent persons about the lower torso.

The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

The term "machine direction (MD)" or "longitudinal" herein refers to a direction running parallel to the maximum linear dimension of the article and/or fastening material and includes directions within ±45° of the longitudinal direction.

The term "cross direction (CD)", "lateral" or "transverse" herein refers to a direction which is orthogonal to the longitudinal direction.

The term "perimeter" herein refers to the outer limits of an area.

The term "field" herein refers to the area which is interior of the perimeter.

The term "liftable portion" herein refers to that portion of a landing zone which is substantially free from adhesive such that it is capable of lifting when a generally z-direction load is applied (e.g., an attempt to disengage a hook from said liftable portion).

The term "% coverage" herein refers to the percent of the knitted fabric within an area or along a line that is bonded to the underlying substrate either by a bonding agent (e.g., adhesive) or fusion bond.

The term "% overall coverage" herein refers to the percent of the knitted fabric within an area that is bonded to the underlying substrate either by a bonding agent (e.g., adhesive) or fusion bond. This term is frequently used to describe a pattern generally.

The term "joined" encompasses configurations whereby an element is directly secured to another element by affixing the element directly to the other element, and configurations whereby an element is indirectly secured to another element by affixing the element to intermediate member(s) which in turn are affixed to the other element.

### Description:

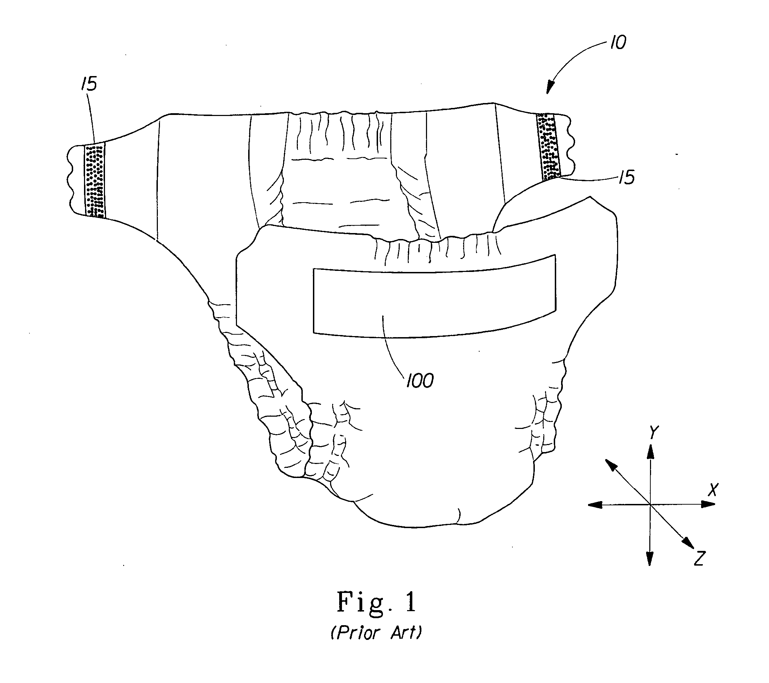

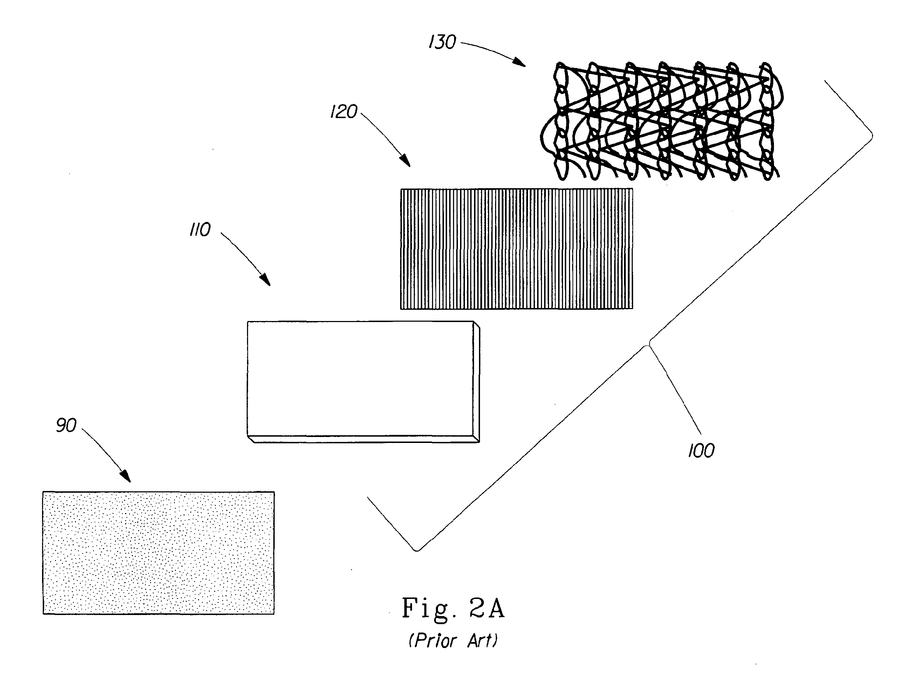

FIG. 1 depicts an exemplary embodiment of a conventional absorbent article 10 having a fastening system comprising a male portion 15 and a female portion 100. The male portion 15 may comprise a plurality of hooks. The female portion 100 may comprise a plurality of loops. FIG. 2a shows an exploded view of an exemplary female portion 100. Said female portion 100 may comprise an underlying substrate layer 110, a construction adhesive layer 120 and a knitted fabric layer 130. Said female portion 100 may be attached to absorbent article 10 by way of a bonding adhesive layer 90. The construction adhesive layer 120 may be applied to underlying substrate 110 and/or knitted fabric layer 130. Underlying substrate 110 may be made of any suitable material including, but not limited to, film, nonwoven, extensible materials, elastomeric materials, cellulosic materials, plastic and combinations thereof.

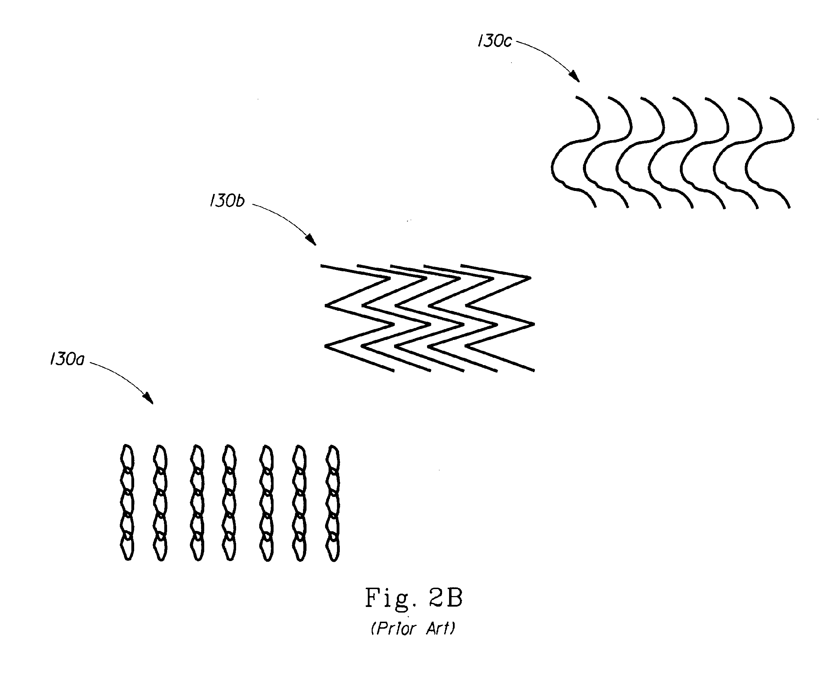

The elements and construction of female portion 100 will be discussed later in more detail. FIG. 2b shows an exploded view of an exemplary knitted fabric layer 130. Said knitted fabric layer 130 may comprise a series of chains 130a, a series of wefts 130b, and a series of loops 130c which may be knitted together.

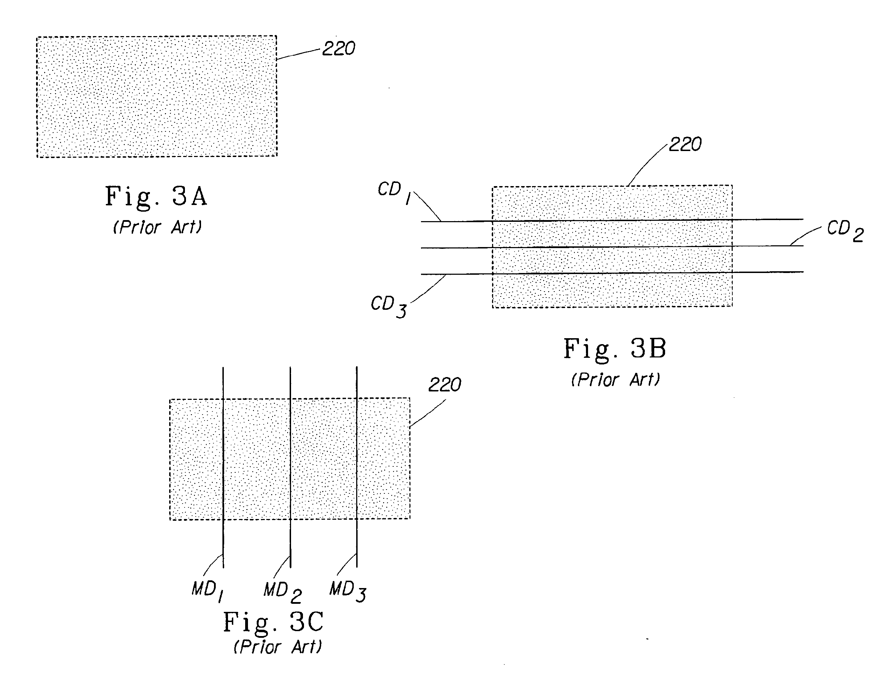

FIG. 3a shows an exemplary print pattern of a construction adhesive layer 220 which is known in the prior art. Construction adhesive layer 220 provides 100% overall coverage and subsequent bonding between the underlying substrate and fabric layer. FIG. 3b shows construction adhesive layer 220 being analyzed along three exemplary lines (CD₁, CD₂ and CD₃) in the CD direction. More specifically, the percent coverage along said lines is analyzed. In this particular embodiment, adhesive is present along the entire length of all three lines such that there is no variation when measured multiple times in the CD direction. As such, the minimum CD coverage is 100% and the maximum CD coverage is 100% with a standard deviation of 0%. Similarly, FIG. 3c shows construction adhesive layer 220 being analyzed along three exemplary lines (MD₁, MD₂ and MD₃) in the MD direction. In this particular embodiment, adhesive is present along the entire lengths of all three lines such that there is no variation when measured multiple times in the MD direction. As such, the minimum MD coverage is 100% and the maximum MD coverage is 100% with a standard deviation of 0%.

FIG. 4a shows an exemplary print pattern of a construction adhesive layer 320a which is known in the prior art. Construction adhesive layer 320a provides about 40% overall coverage and subsequent bonding between the substrate and fabric layer. More specifically, construction adhesive layer 320a is shown having vertical lines of adhesive in the MD direction with vertical spacing 310a therebetween. FIG. 4b shows construction adhesive layer 320a being analyzed along three exemplary lines (CD₁, CD₂ and CD₃) in the CD direction. More specifically, the percent coverage along said lines is analyzed. In this particular embodiment, the presence of adhesive appears and disappears along the entire length of all three CD lines in a repeating pattern. As such, the minimum CD coverage is about 40% and the maximum CD coverage is about 40% with a standard deviation of about 0%. Similarly, FIG. 4c shows construction adhesive layer 320a being analyzed along three exemplary lines (MD₁, MD₂ and MD₃) in the MD direction. In this particular embodiment, adhesive is present along the entire length of some of the lines and not present along the entire length of the remaining lines. As such, the minimum MD coverage is about 0% and the maximum MD coverage is about 100% with a standard deviation of about 49%.

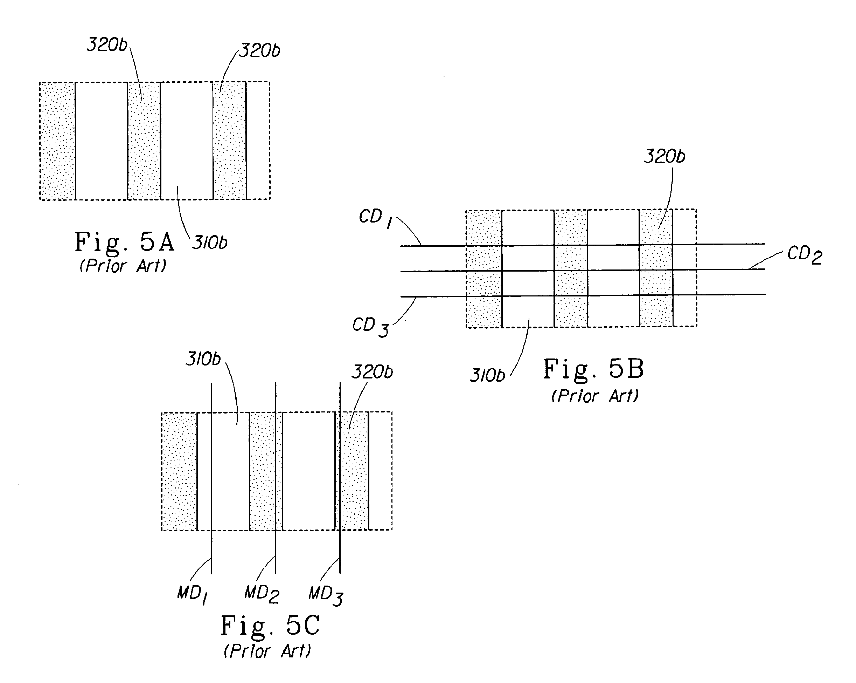

FIG. 5a shows an exemplary print pattern of a construction adhesive layer 320b which is known in the prior art. Construction adhesive layer 320b provides about 40% overall coverage and subsequent bonding between the underlying substrate and fabric layer. More specifically, construction adhesive layer 320b is shown having vertical lines of adhesive in the MD direction with vertical spacing 310b therebetween. The vertical spacing 310b in FIG. 5a is greater than the vertical spacing 310a in FIG. 4a. FIG. 5b shows construction adhesive layer 320b being analyzed along three exemplary lines (CD₁, CD₂ and CD₃) in the CD direction. More specifically, the percent coverage along said lines is analyzed. In this particular embodiment, the presence of adhesive appears and disappears along the entire lengths of all three CD lines in a repeating pattern. As such, the minimum CD coverage is about 40% and the maximum CD coverage is about 40% with a standard deviation of about 0%. Similarly, FIG. 5c shows construction adhesive layer 320b being analyzed along three exemplary lines (MD₁, MD₂ and MD₃) in the MD direction. In this particular embodiment, adhesive is present along the entire length of some of the lines and not present along the entire length of the remaining lines. As such, the minimum MD coverage is about 0% and the maximum MD coverage is about 100% with a standard deviation of about 49%.

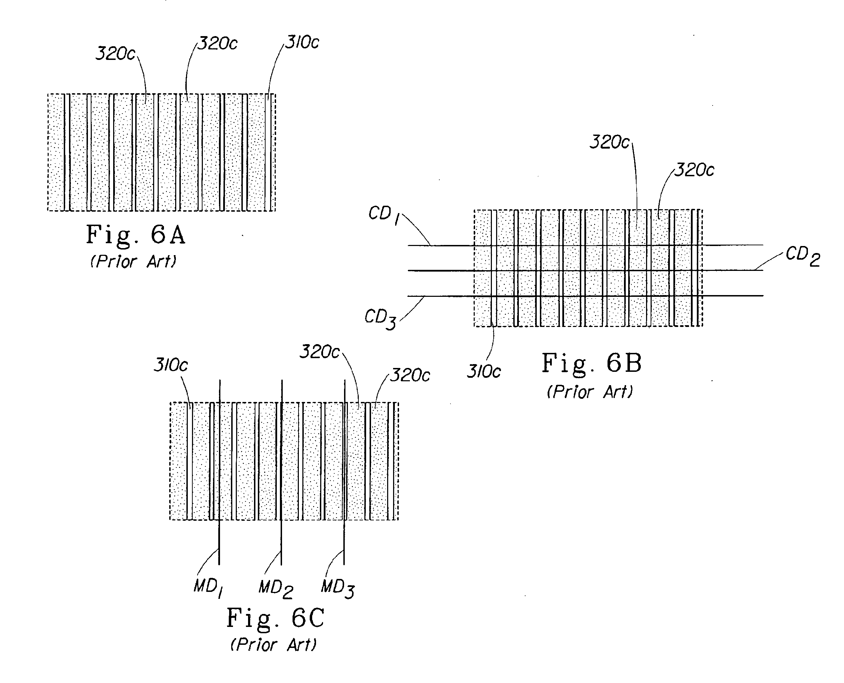

FIG. 6a shows an exemplary print pattern of a construction adhesive layer 320c which is known in the prior art. Construction adhesive layer 320c provides about 80% overall coverage and subsequent bonding between the substrate and fabric layer. More specifically, construction adhesive layer 320c is shown having vertical lines of adhesive in the MD direction with vertical spacing 310c therebetween. The vertical spacing 310c is the narrowest among the entire prior art examples illustrated herein. FIG. 6b shows construction adhesive layer 320c being analyzed along three exemplary lines (CD₁, CD₂ and CD₃) in the CD direction. More specifically, the percent coverage along said lines is analyzed. In this particular embodiment, the presence of adhesive appears and disappears along the entire lengths of all three CD lines in a repeating pattern. As such, the minimum CD coverage is about 80% and the maximum CD coverage is about 80% with a standard deviation of about 0%. Similarly, FIG. 6c shows construction adhesive layer 320c being analyzed along three exemplary lines (MD₁, MD₂ and MD₃) in the MD direction. In this particular embodiment, adhesive is present along the entire length of some of the lines and not present along the entire length of the remaining lines. As such, the minimum MD coverage is about 0% and the maximum MD coverage is about 100% with a standard deviation of about 40%.

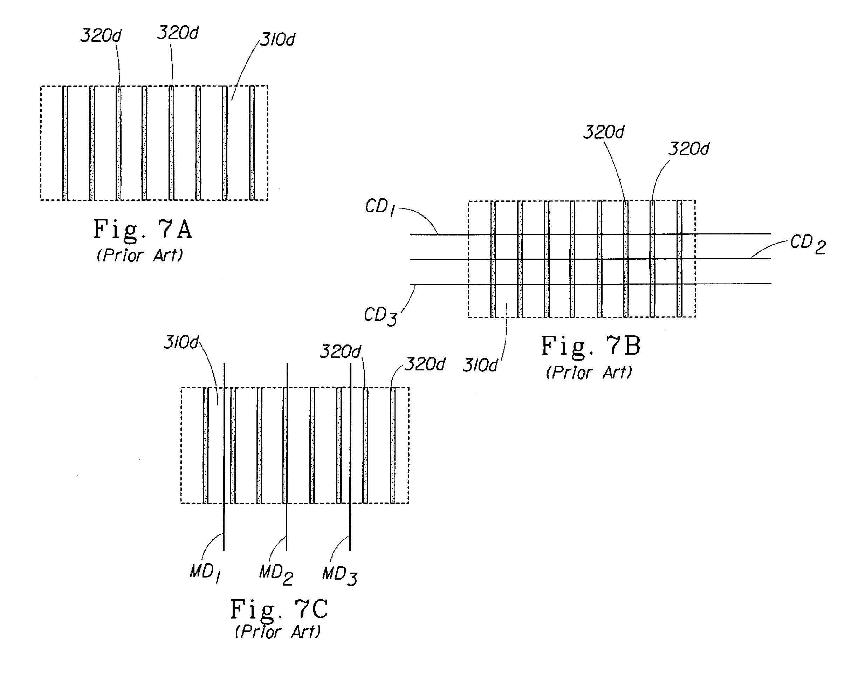

FIG. 7a shows an exemplary print pattern of a construction adhesive layer 320d which is known in the prior art. Construction adhesive layer 320d provides about 14% overall coverage and subsequent bonding between the substrate and fabric layer. More specifically, construction adhesive layer 320d is shown having vertical lines of adhesive in the MD direction with vertical spacing 310d therebetween. The construction adhesive layer 320d is the narrowest among the entire prior art examples illustrated herein. FIG. 7b shows construction adhesive layer 320d being analyzed along three exemplary lines (CD₁, CD₂ and CD₃) in the CD direction. More specifically, the percent coverage along said lines is analyzed. In this particular embodiment, the presence of adhesive appears and disappears along the entire lengths of all three CD lines in a repeating pattern. As such, the minimum CD coverage is about 14% and the maximum CD coverage is about 14% with a standard deviation of about 0%. Similarly, FIG. 7c shows construction adhesive layer 320c being analyzed along three exemplary lines (MD₁, MD₂ and MD₃) in the MD direction. In this particular embodiment, adhesive is present along the entire length of some of the lines and not present along the entire length of the remaining lines. As such, the minimum MD coverage is about 0% and the maximum MD coverage is about 100% with a standard deviation of about 35%.

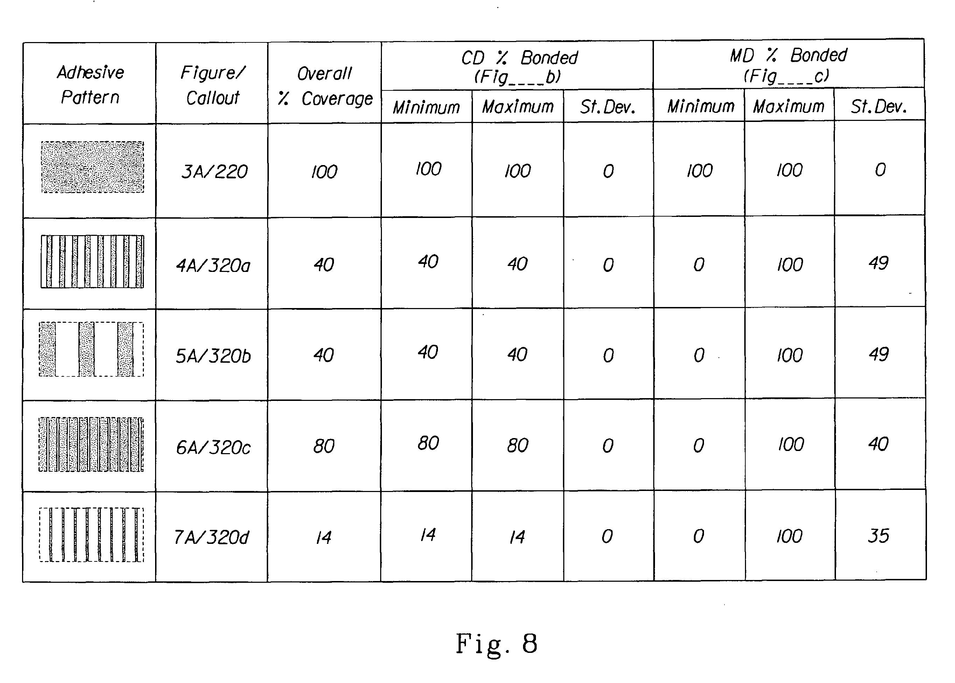

FIG. 8 shows a chart of the prior art construction adhesive layers discussed above. While the data has already been discussed above, it is important to note that none of these five print patterns have both a CD and MD standard deviation which are not equal to zero. The importance of such fact will be discussed later.

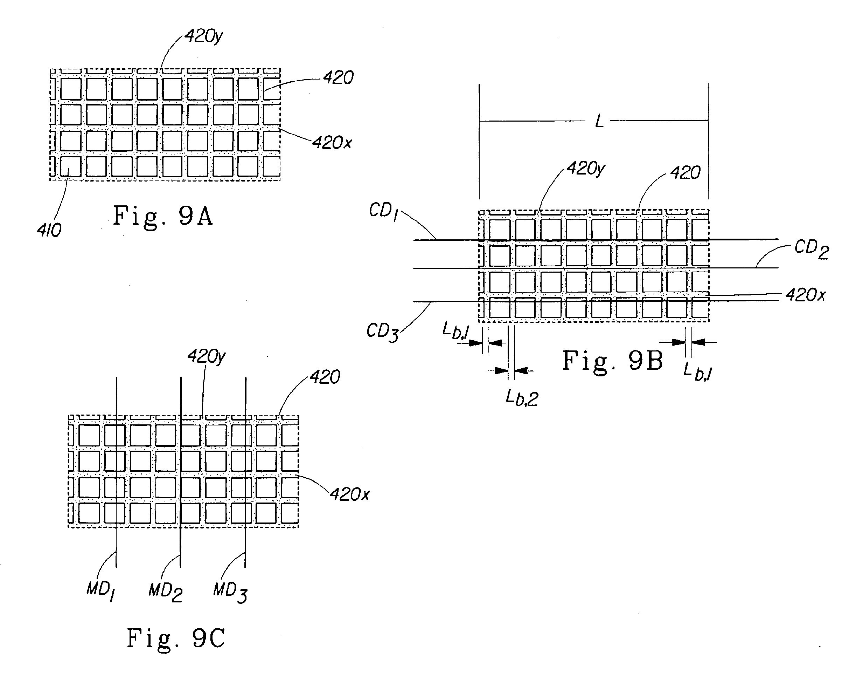

FIG. 9a shows an exemplary print pattern of a construction adhesive layer 420 in accordance with the present invention. Construction adhesive layer 420 provides about 40% coverage and subsequent bonding between the substrate and fabric layer. More specifically, construction adhesive layer 420 is shown having a first plurality of non-intersecting continuous bond lines 420x and a second plurality of non-intersecting continuous bond lines 420y are combined to form a pattern of intersecting bond lines that define tessellating pattern elements 410 that may have equal size and shape. The tessellating pattern elements within this exemplary embodiment are substantially square-shaped. FIG. 9b shows construction adhesive layer 420 being analyzed along three exemplary lines (CD₁, CD₂ and CD₃) in the CD direction. More specifically, the percent coverage along said lines is analyzed. In this particular embodiment, the presence of adhesive is not necessarily the same along all three CD lines. As such, the minimum CD coverage is about 23% and the maximum CD coverage is about 100% with a standard deviation of about 32%. Similarly, FIG. 9c shows construction adhesive layer 420 being analyzed along three exemplary lines (MD₁, MD₂ and MD₃) in the MD direction. In this particular embodiment, the presence of adhesive is not necessarily the same along all three MD lines. As such, the minimum CD coverage is about 23% and the maximum CD coverage is about 100% with a standard deviation of about 32%.

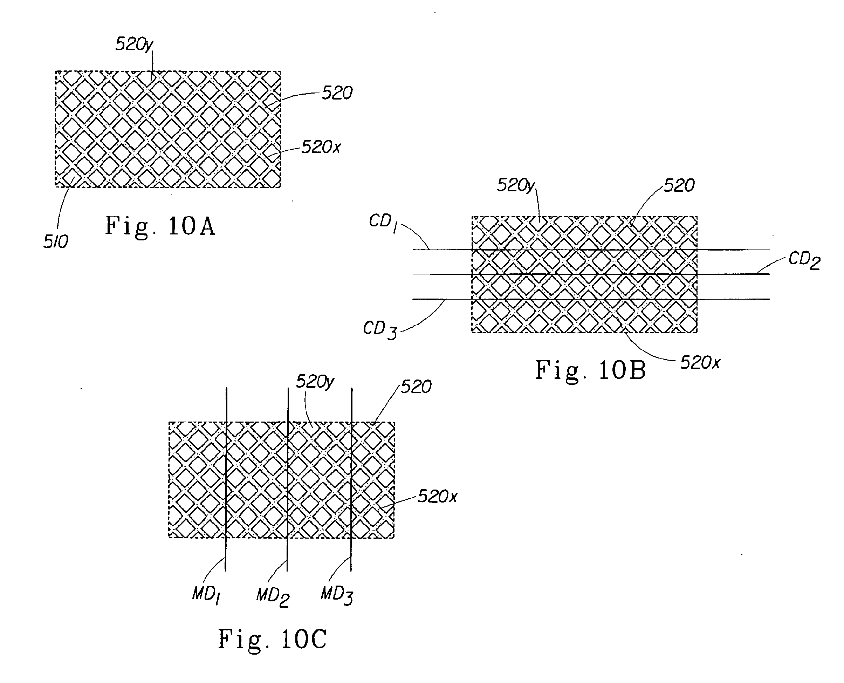

FIG. 10a shows an exemplary print pattern of a construction adhesive layer 520 in accordance with the present invention. Construction adhesive layer 520 provides about 40% coverage and subsequent bonding between the substrate and fabric layer. More specifically, construction adhesive layer 520 is shown having a first plurality of non-intersecting continuous bond lines 520x and a second plurality of non-intersecting continuous bond lines 520y are combined to form a pattern of intersecting bond lines that define tessellating pattern elements 510 that may have equal size and shape. The tessellating pattern elements within this exemplary embodiment are substantially square-shaped which are rotated. FIG. 10b shows construction adhesive layer 520 being analyzed along three exemplary lines (CD₁, CD₂ and CD₃) in the CD direction. More specifically, the percent coverage along said lines is analyzed. In this particular embodiment, the presence of adhesive is not necessarily the same along all three CD lines. As such, the minimum CD coverage is about 23% and the maximum CD coverage is about 46% with a standard deviation of about 7%. Similarly, FIG. 10c shows construction adhesive layer 520 being analyzed along three exemplary lines (MD₁, MD₂ and MD₃) in the MD direction. In this particular embodiment, the presence of adhesive is not necessarily the same along all three MD lines. As such, the minimum CD coverage is about 23% and the maximum CD coverage is about 46% with a standard deviation of about 7%.

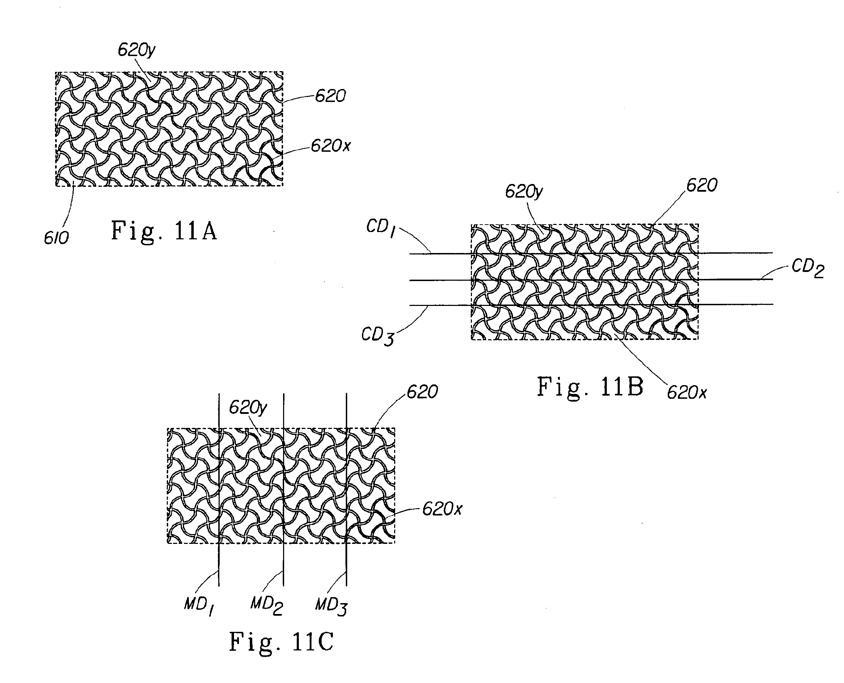

FIG. 11a shows an exemplary print pattern of a construction adhesive layer 620 in accordance with the present invention. Construction adhesive layer 620 provides about 40% coverage and subsequent bonding between the substrate and fabric layer. More specifically, construction adhesive layer 620 is shown having a first plurality of non-intersecting continuous, wavy bond lines 620x and a second plurality of non-intersecting continuous, wavy bond lines 620y are combined to form a pattern of intersecting bond lines that define tessellating pattern elements 610 that may have equal size and shape. The tessellating pattern elements within this exemplary embodiment may be described as substantially "dog-bone". FIG. 11b shows construction adhesive layer 620 being analyzed along three exemplary lines (CD₁, CD₂ and CD₃) in the CD direction. More specifically, the percent coverage along said lines is analyzed. In this particular embodiment, the presence of adhesive is not necessarily the same along all three CD lines. As such, the minimum CD coverage is about 31% and the maximum CD coverage is about 61% with a standard deviation of about 10%. Similarly, FIG. 11c shows construction adhesive layer 620 being analyzed along three exemplary lines (MD₁, MD₂ and MD₃) in the MD direction. In this particular embodiment, the presence of adhesive is not necessarily the same along all three MD lines. As such, the minimum CD coverage is about 31% and the maximum CD coverage is about 61% with a standard deviation of about 10%.

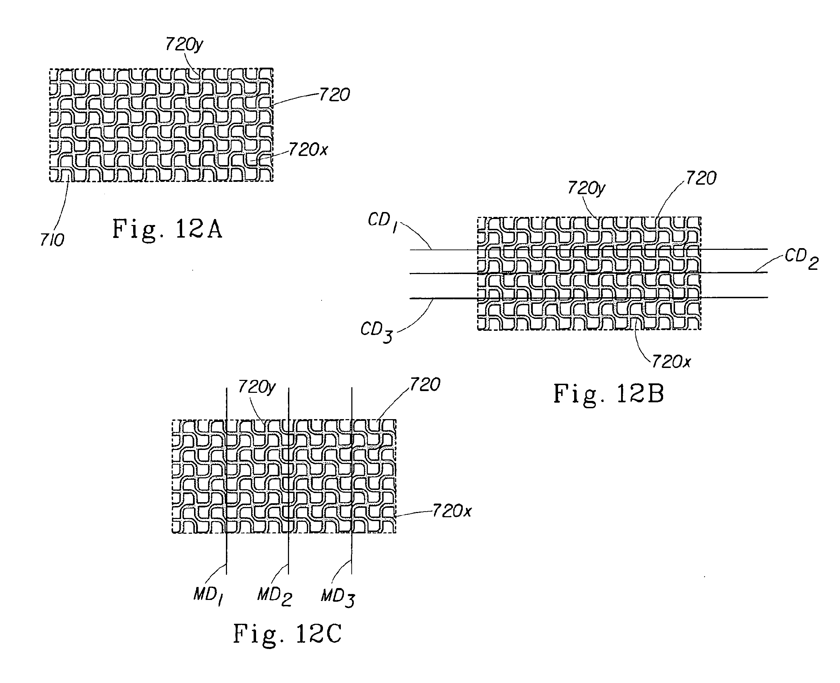

FIG. 12a shows an exemplary print pattern of a construction adhesive layer 720 in accordance with the present invention. Construction adhesive layer 720 provides about 40% coverage and subsequent bonding between the substrate and fabric layer. More specifically, construction adhesive layer 720 is shown having a first plurality of non-intersecting continuous, wavy bond lines 720x and a second plurality of non-intersecting continuous, wavy bond lines 720y are combined to form a pattern of intersecting bond lines that define tessellating pattern elements 710 that may have equal size and shape. The tessellating pattern elements within this exemplary embodiment may be described as substantially "dog-bone". FIG. 11b shows construction adhesive layer 720 being analyzed along three exemplary lines (CD₁, CD₂ and CD₃) in the CD direction. More specifically, the percent coverage along said lines is analyzed. In this particular embodiment, the presence of adhesive is not necessarily the same along all three CD lines. As such, the minimum CD coverage is about 24% and the maximum CD coverage is about 89% with a standard deviation of about 24%. Similarly, FIG. 11c shows construction adhesive layer 720 being analyzed along three exemplary lines (MD₁, MD₂ and MD₃) in the MD direction. In this particular embodiment, the presence of adhesive is not necessarily the same along all three MD lines. As such, the minimum CD coverage is about 24% and the maximum CD coverage is about 89% with a standard deviation of about 24%.

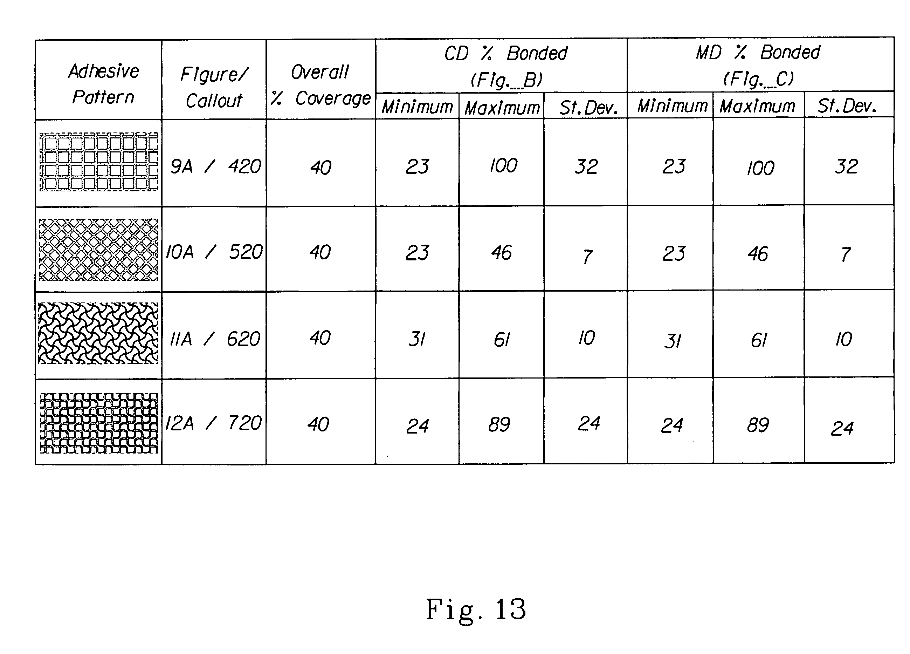

FIG. 13 shows a chart of the novel construction adhesive layers discussed above. While the data has already been discussed above, it is important to note that all four of novel print patterns have both a CD and MD standard deviation which are not equal to zero. It has been discovered that having both a CD and MD standard deviation which are not equal to zero is critical for ensuring sufficient bonding along the perimeter while providing sufficient unbonded, liftable portions within the field so as to satisfy the competing interests of structural integrity, connection performance and frayed edges.

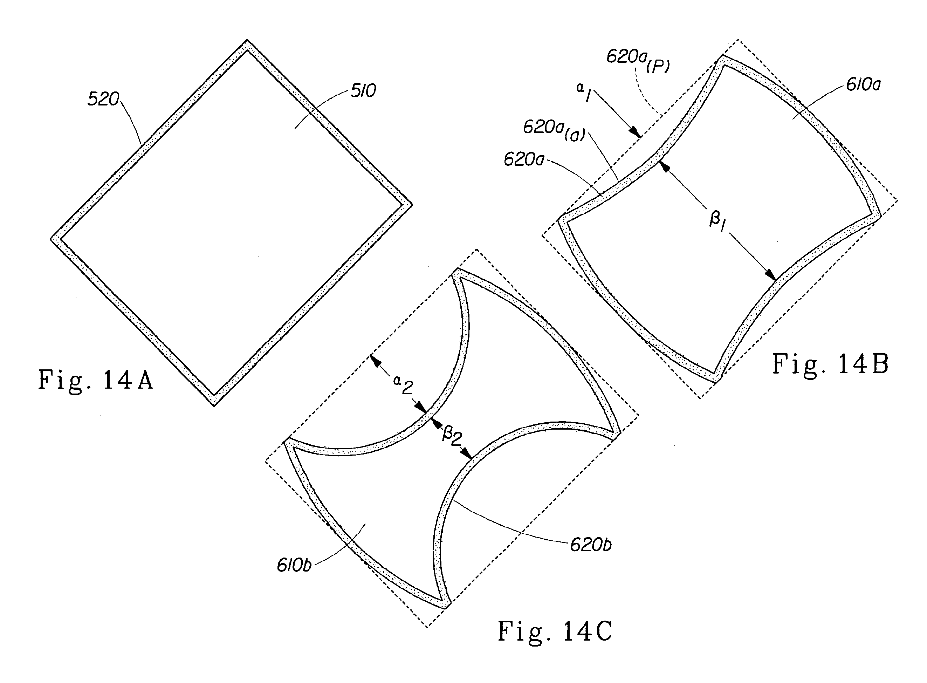

FIG. 14a shows an exemplary portion of an adhesive print pattern in accordance with the present invention. More specifically, tessellating pattern element 520 is formed by intersecting lines of adhesive which form an interior pattern portion 510 that serves as a liftable portion during use. Tessellating pattern element 520 is similar to those cells depicted in FIG. 10a in that its side edges are substantially linear. Now referring to FIG. 14b, tessellating pattern element 620a is shown with nonlinear side edges. An amplitude, α₁, is shown as a maximum distance measured from a projected linear side edge 620a₍ₚ₎ to the actual nonlinear side edge 620a₍ₐ₎. Said maximum distance need not be located along the mid-point of said sides. A distance or gap between the side edges is measured as β₁, wherein β₁ may be measured between any two opposing side edges. Now referring to FIG. 14c, interior pattern portion 620b is shown having an amplitude, α₂, which is larger than α₁ in FIG. 14b, such that, the corresponding gap β₂ is smaller than β₁. Increasing the amplitude, as exampled in FIG. 14b, will increase the standard deviation of percent coverage, thus attempting to further address the competing interests of structural integrity, connection performance and frayed edges. However, increasing amplitude will also decrease the gap, β, which decreases the area of liftable portion. Further, if adhesive is used, decreasing the gap, β, may lead to adhesive contamination between the side edges as a result of possible process variations. Such adhesive contamination may result in the undesirable creation of two distinct interior pattern portions having different performance characteristics from that of the rest of the fabric. Additionally, such adhesive contamination may result in the artificially increasing of the overall percent coverage which may lead to lower connection performance since the hooks would have fewer unglued loops available for connection. Further, gap β should not be too large, otherwise, the risk of frayed edges may increase.

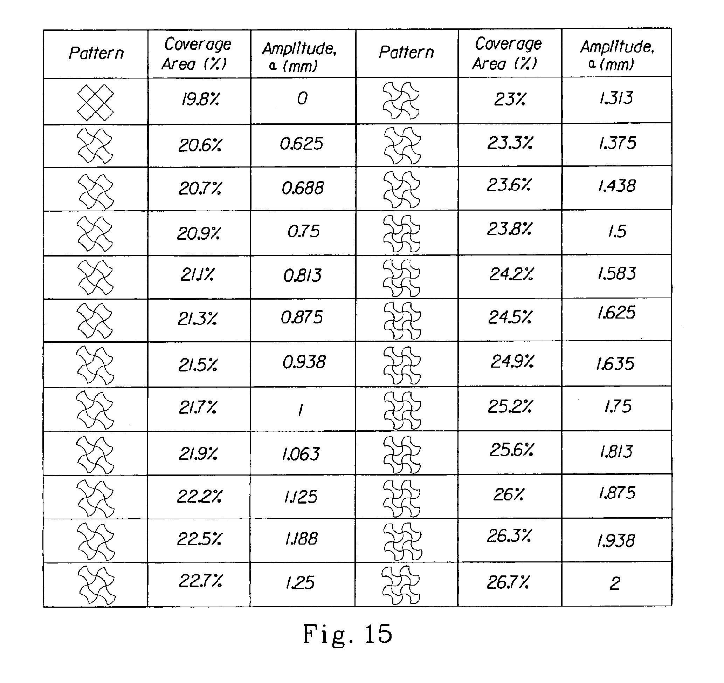

Fig.15 shows a chart of exemplary adhesive patterns having varying percent coverage areas and varying amplitudes. Given the relationships discussed above, one skilled in the art could select an appropriate pattern for a particular product design, more specifically, for a particular knitted fabric structure and/or lamination process.

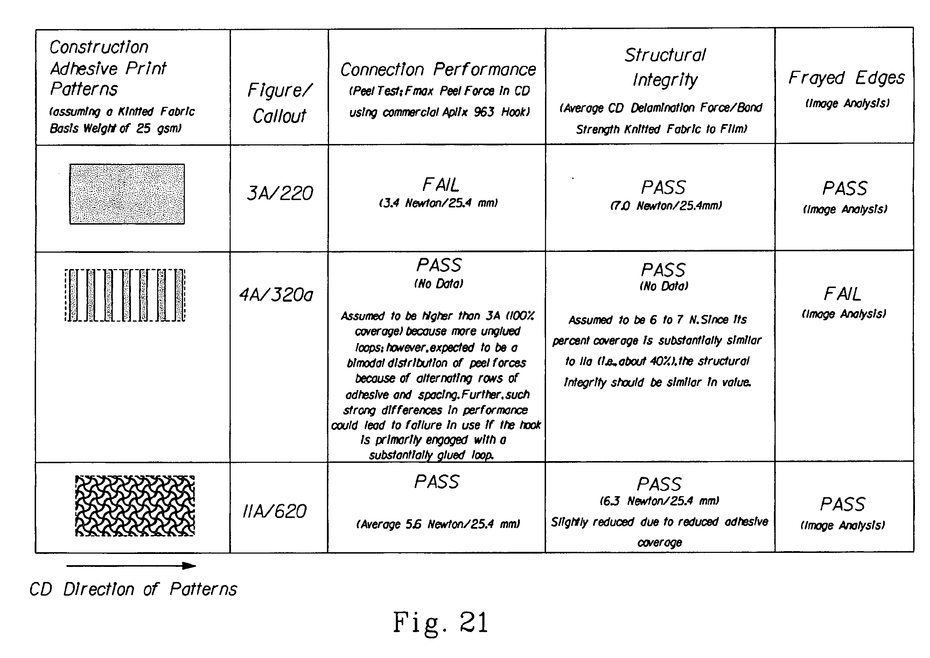

Referring to pattern variations in Fig. 15 (assuming all having an equal line width of 0.58 mm), in the manufacture of a female fastening portion for a disposable absorbent article, it may be desirable to have an amplitude being greater than 0 to about 3 mm and a standard deviation for both MD and CD % bonded being greater than 0 to about 32%. Further, it may be desirable to have a gap from about 2mm to about 5mm. One such exemplary embodiment may include: Knitted fabric from Nylon Knitting, Malta (code of 2699/64) having a basis weight of about 25 gsm, having a bond pattern similar to FIG. 11a, having a line width of about 2.3 mm, having a total adhesive coverage of about 40%, having a minimum gap of about 6 mm with a corresponding amplitude of about 2 mm, and having a standard deviation in both MD and CD % bonded of about 10%. The resulting benefits are shown as being a "PASS" in the chart of FIG. 21.

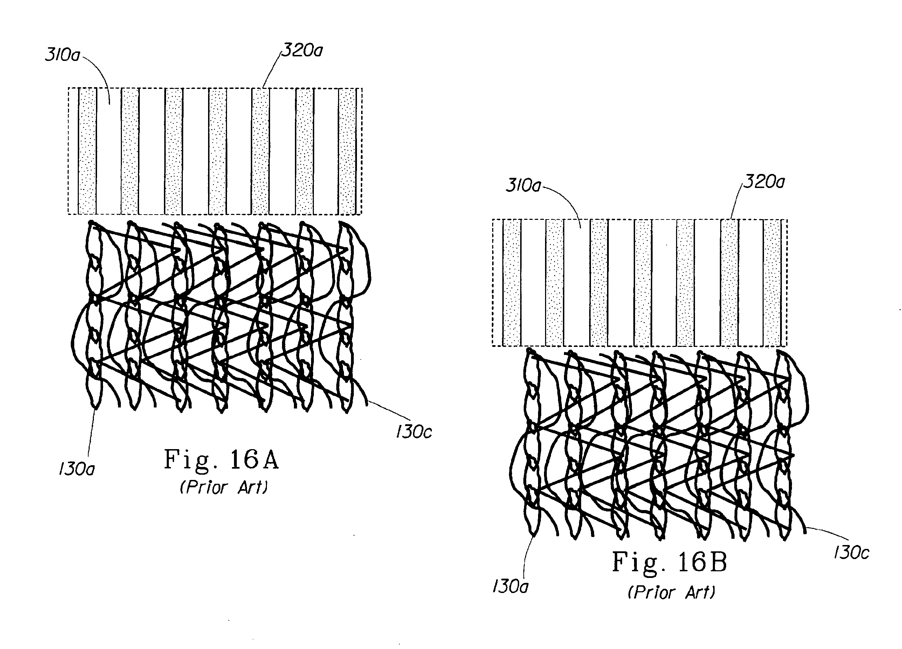

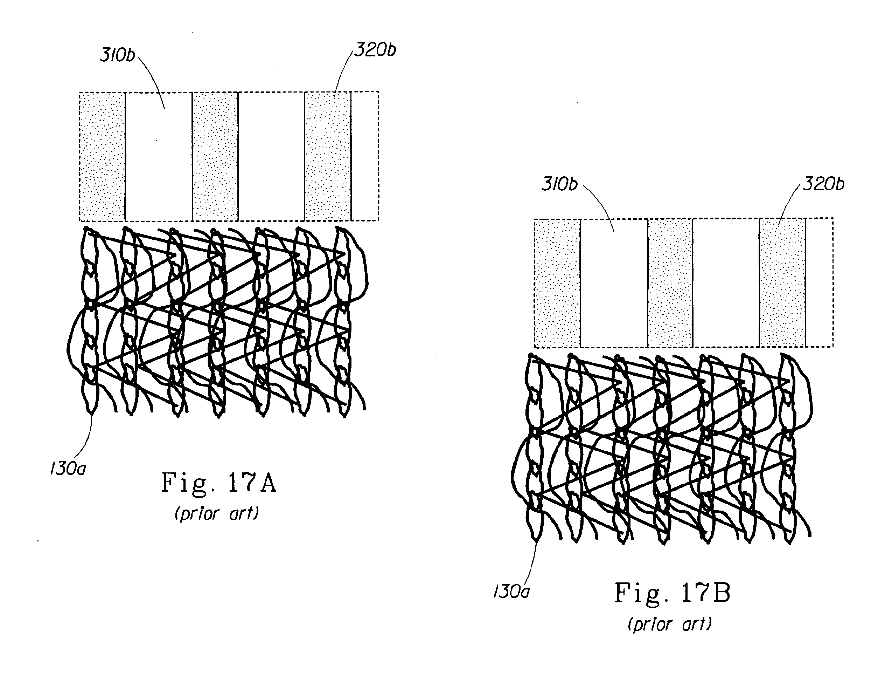

FIG. 16a shows an exemplary knitted fabric and an underlying print pattern which are known in the prior art. When using knitted fabrics, it is often intended within the prior art to align and/or register the lines of adhesive with chains 130a so as to substantially adhere the chains to the underlying substrate while not substantially adhering the loops 130c. However, maintaining proper alignment is often difficult. Such misalignment is illustrated in FIG. 16b wherein the adhesive is more aligned with the loops and wefts, rather than with the chains. One known attempt in the prior art to correct for such misalignment is to substantially widen the width of the lines of adhesive 320b, as exampled in FIG. 17a. In doing so, the lines of adhesive 320b are sufficiently wide so as to adhere at least one series of chains despite any potential misalignment (see FIG. 17b). However, increasing the line width in this way results in an increased gap width which may result in an increased risk of frayed edges. As such, this prior art attempt does not meet all the requirements as set forth in the present invention.

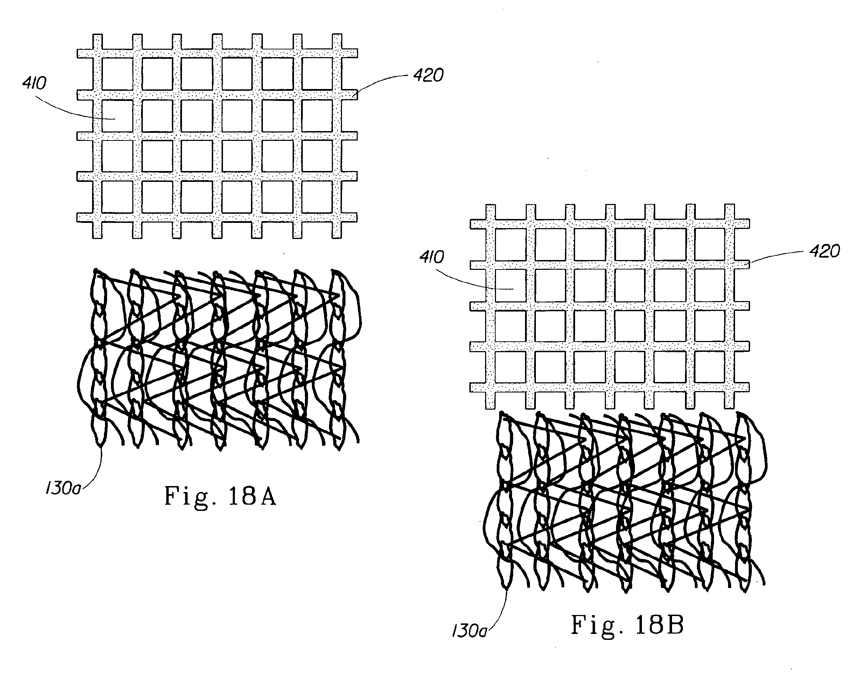

The novel print patterns of the present invention, as exampled in FIG. 18a, provide lines of adhesive 420 in both the CD and MD direction such that any potential misalignment, as exampled in FIG. 18b, does not present any significant problems. More specifically, even if the MD lines of adhesive 420y are misaligned with the chains 130a so as not to adhere to them, the CD lines of adhesive 420x will still intersect with said chains. Furthermore, because the CD and MD lines form tessellating pattern elements having interior pattern portions, there still exists open area without adhesives for the loops to remain substantially free from adhesive. Consequently, said interior pattern portions create liftable portions for said loops.

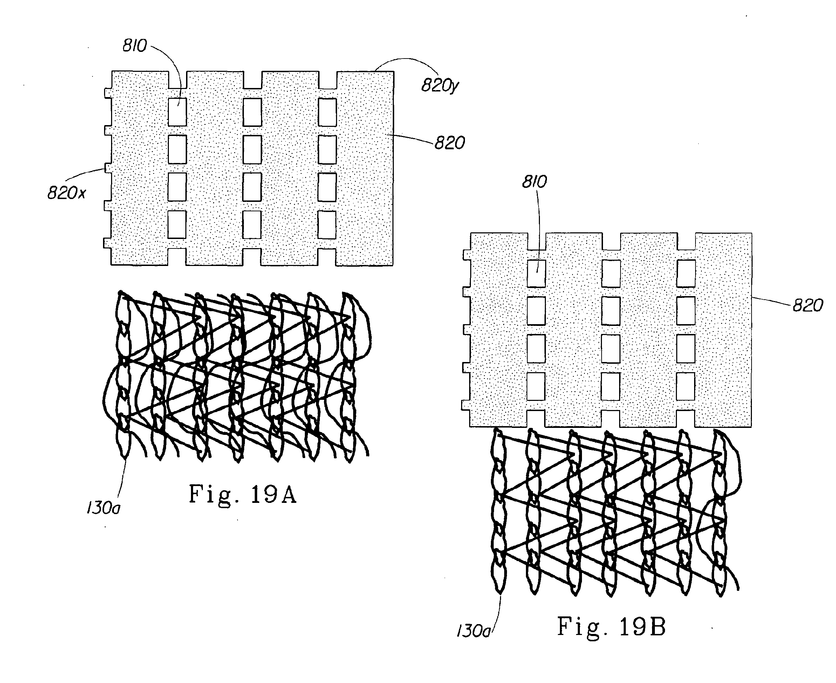

As used herein, a bond line (or line of adhesive) is preferably a linear construction having a constant width, or line "weight". However, it is recognized that lines having substantially different widths are sufficient for the benefits of the present invention. Therefore, while a pattern of lines having a constant width may be preferred, those skilled in the art will recognize that much of the benefit of the present invention can be achieved by the use of lines having a varying width. For example, FIG. 19a shows lines of adhesive 820y in the MD direction being wider than lines of adhesive 820x in the CD direction. In this particular embodiment, however, it should be appreciated that the percent coverage area may correspondingly increase and such fact should be considered within the product design.

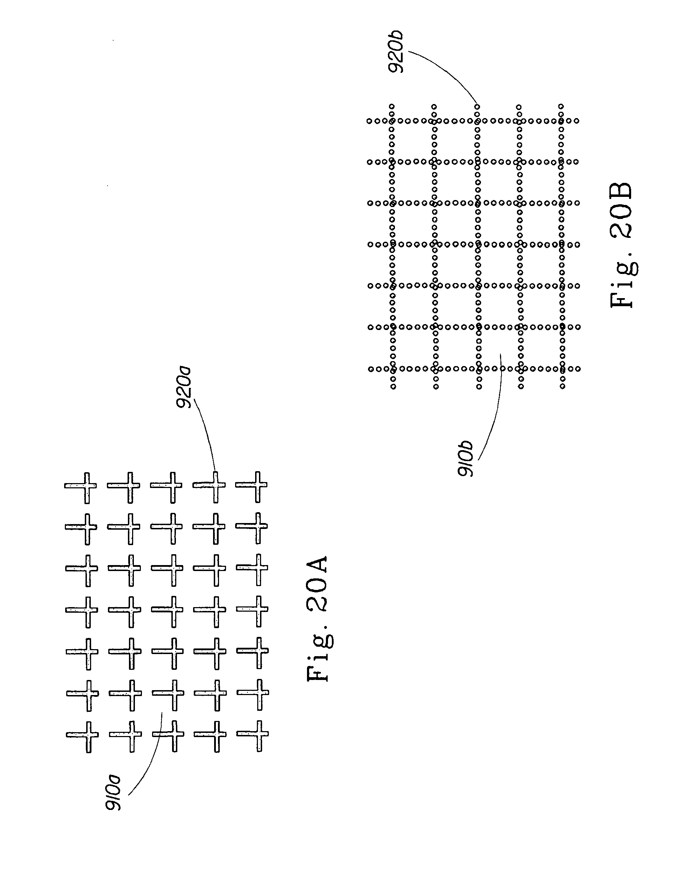

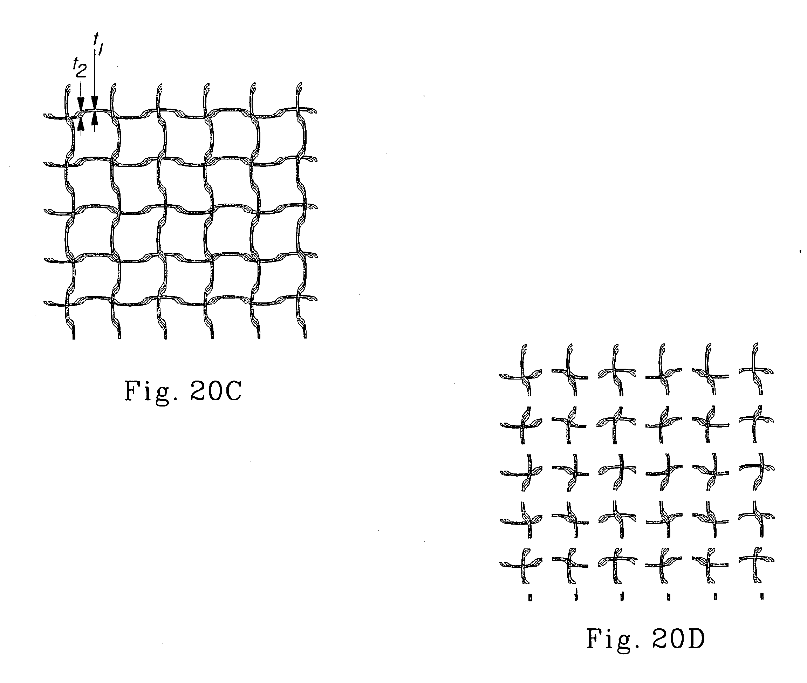

While the pattern of the present invention is disclosed as intersecting bond "lines", it is recognized that the term "line" can also describe a series of discrete points or broken lines so closely spaced as to effectively approximate a line. For example, FIG. 20a shows a novel print pattern having broken lines. In another example, FIG. 20b shows a novel print pattern having discrete points or dots. In yet another example, FIG. 20c shows a novel print pattern having non-linear lines. Further, FIG. 20c demonstrates that the lines themselves may have differing thickness (e.g., t₁ > t₂). In yet another example, FIG. 20d shows a novel print pattern having non-linear, broken lines.

FIG. 21 shows a chart which further demonstrates the novel aspects of the present invention. Said chart provides actual and theoretical data for the tests of connection performance, structural integrity and frayed edges. Where actual data is shown, the following materials were used: Aplix Hook 963 (commercially available from Aplix, Z.A. Les Relandieres - RN 23, 44850 Le Cellier, France) and Landing Zone (CD: 13mm x MD: 25.4mm dimension; laminated to a printed film using polyamide knitted fabric at 25 gsm basis weight code: 2699/64; laminating by Nordenia, Jöbkesweg 11, 48559 Gronau, Germany and polyamide fabric from Nylon Knitting LTD, Manwel Dimech Str., Qormi QRM 11, Malta).

As already discussed in the background, providing a print pattern which meets all three test method requirements is difficult because of their competing interests (i.e., connection performance prefers less adhesive; structural integrity prefers more adhesive; and frayed edges prefers more adhesive particularly around the perimeter). The first row in said chart provides the corresponding data for an about 100% coverage print pattern, wherein connection performance fails due to too many loops being glued (i.e., the peel / fastening performance is significantly reduced). The second row in said chart provides the corresponding data for a vertically-striped print pattern having about 40% coverage, wherein frayed edges fails due to loose edges (for example, left side of print pattern) and large gaps/spacing between the stripes. [Since a physical sample of said vertical-striped was not immediately available, the PASS results were assumed]. The third row in said chart illustrates the provides the corresponding data for an about 40% coverage print pattern in accordance with the present invention, wherein all three test methods having a PASS value.

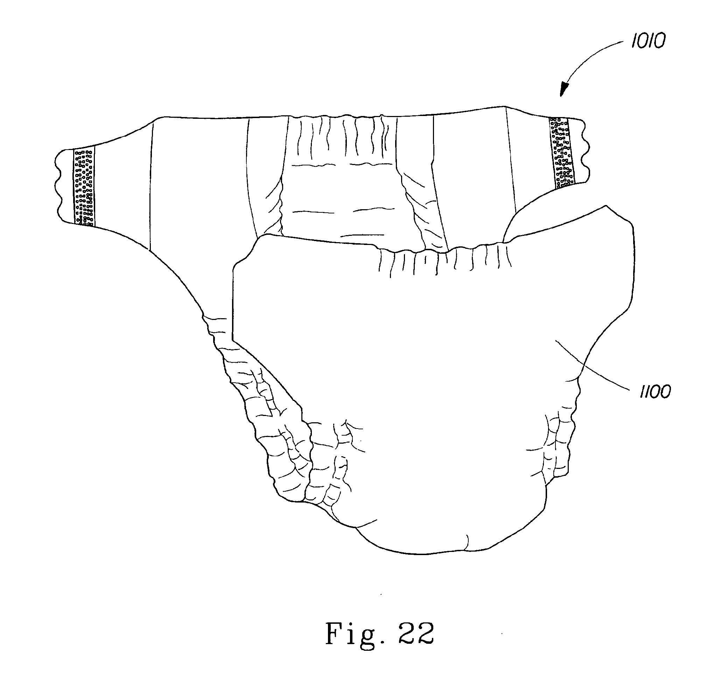

In contrast to FIG. 1, FIG. 22 shows an alternative embodiment of an absorbent article 1010 wherein the landing zone (1010) substantially covers the entire surface of said absorbent article.

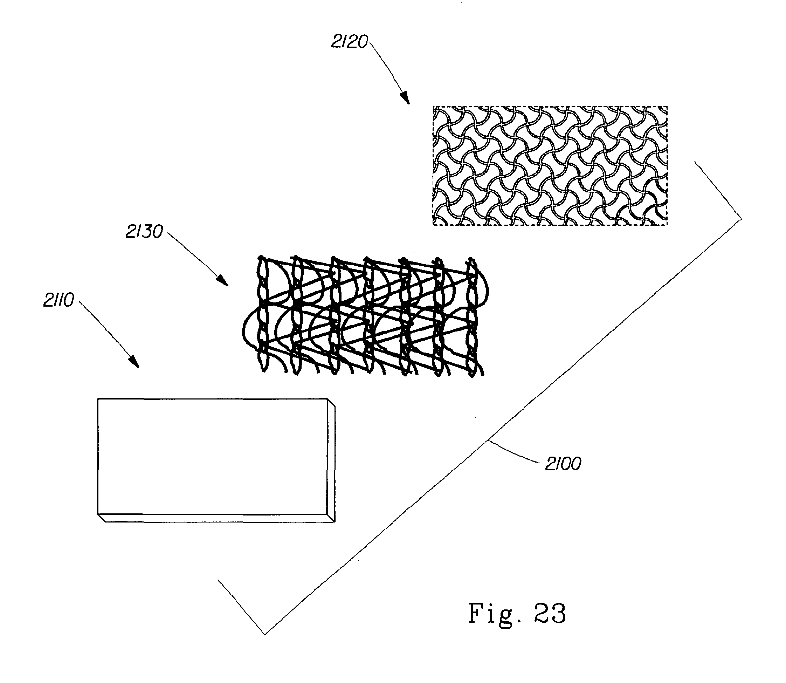

As an alternative to the general construction depicted in FIG. 2, FIG. 23 depicts an exemplary embodiment of the present invention wherein female portion 2100 may comprise an underlying substrate 2110 and a knitted fabric layer 2130 which are fusion bonded together using bonding pattern 2120, rather than using a bonding agent (e.g., adhesive). Such fusion bonding may be accomplished by using known technologies including, but not limited to, thermal bonding, pressure bonding, ultrasonic bonding, radio frequency bonding and combinations thereof.

### TEST METHODS

Percent coverage and percent overall coverage may be measured and calculated using industry-standard methods, such as computerized image analysis or by merely physically measuring the sample (for example, using a ruler, digital calipers, or other suitably calibrated measurement device). For example, one can visually identify areas in which bonding between the knit fabric & underlying layer are present & areas which bonding is not present. Percent coverage for a given cut orientation (CD or MD) along a length of a line is determined as follows:
1. Measure, to at least the nearest 0.5 mm, the total length of the line being measured (defined as "L"; e.g., see FIG. 9b).
2. Measure, to at least the nearest 0.5 mm, the length of each individual bonded (L_{b,i}) portion along the line (e.g., see FIG. 9b).
3. Determine the total length bonded within the line by summing all the individual lengths bonded (L_{b}= sum of all L_{b,i}) along the line being measured. Note that some bond patterns will have multiple individual lengths of bonded portions to measure individual lines (as in Line CD₁ of Figure 9b) or a single length to measure, as in Line CD₂ of Figure 9b.
4. Calculate % Coverage for the line measured (% Coverage = 100 * L_{b}/L).
5. To determine standard deviation of % coverage, steps 1-3 are completed for multiple lines (CDᵢ and MDᵢ). While herein the CD and MD test measurements refer to three exemplary lines of measurement, as many data points are to be collected as possible. To do this, Lines are measured starting at a first perimeter edge, then in no greater than 1 mm increments from that edge to the opposing perimeter edge. Standard deviation is calculated via standard statistical analysis calculations from the multiple measures of % coverage obtained for the various lines measured.

Whenever possible and practical, it is recommended to perform computerized image analysis to determine pattern measurements. The % coverage data contained herein was all obtained using such an analysis. In computerized image analysis, one digitizes an image of the pattern that contains the bonded and unbonded areas in a manner in which a color contrast can be reliably measured to determine where bonded and unbonded areas are present. For example, unbonded areas may be represented as white pixels & bonded areas may be represented as black pixels. Preferably, the pixel density is at least 6 pixels/mm. As many lines as possible are nneasured - preferably, individual Lines are just 1 pixel in width. Number of pixels representing bonded lengths (or areas) can be counted and compared to number of pixels representing total line length (or total area) to calculate % coverage for lines and/or for areas. Pixels counts may be converted to actual lengths or may be used directly if only relative (percentage) measures are needed.

Amplitude (α) and gap (β) can also be measured using physical measurements or image analysis techniques. All measurements are completed on samples which have been conditioned for 24 hours in controlled conditions (Temperature = 23 ± 2 °C, Humidity = 50 ± 5 % RH).

### PRODUCT APPLICATIONS

One skilled in the art would appreciate that the bonds patterns and techniques of the present invention may be used in a multitude of applications including, but not limited to, disposable absorbent articles, body wraps, clothing, packaging, feminine hygiene products, bandages, bibs, food wraps, abrasive systems, cleaning systems and polishing systems. However, it is particularly beneficial for disposable absorbent articles, more specifically with respect to its use in the construction of a fastener.

For example, while the knitted fabric discussed herein contain three different types of yarns (i.e., chains, wefts, and loops), one skilled in the art would appreciate that a variety of number and types of yarns, as well as yarns having varying number of filaments, may be practiced within the scope of the present invention. For instance, the knitted fabric may consist primarily of 2 yarns instead of 3 yarns (e.g., yarns of wefts and yarns of chains without the presence of loops, hereinafter, "loop-less knit fabric"). Use or a loop-less knit fabric would provide a significant cost reduction because of its reduced basis weight via the elimination of said loops. When the novel bonding patterns of the present invention are used in conjunction with loop-less knit fabric, the interior pattern portions (i.e., areas without glue) provide unglued areas available for male hook engagement. This unique combination of the novel bonding patterns and loop-less knit fabric provides a cheaper, and more importantly, functional product design having sufficient structural integrity and connection performance.

For example, while particular pattern characteristics have been discussed, one skilled in the art would appreciate that such characteristics may be varied and still fall within the scope of the present invention. For instance, the overall % coverage may range from about 5% to about 90%, more preferably from about 20% to about 70%, and most preferably from about 30% to about 45%. Similarly, the line width may range from about 0.1 mm to about 10 mm, more preferably from about 0.5 mm to about 4 mm, and most preferably from about 1.0 mm to 2.5 mm. Similarly, the amplitude may range from about 0 mm to about 10 mm, more preferably greater than 0 mm to about 6 mm, and most preferably greater than 0 mm to about 3 mm. Similarly, the gap may range from about 0.1 mm to about 15 mm, more preferably from about 0.5 mm to about 7 mm, most preferably from about 2 mm to about 6 mm. Similarly, the standard deviation of % coverage in MD and CD directions may range from about 1% to about 99 %, more preferably from about 5% to about 50%, most preferably from about 5% to 35%. Similarly, the minimum % bonded in the MD and CD directions may be greater than 10%, more preferably greater than 20%, most preferably greater than 30%.

Furthermore, in an effort to optimize a particular bond pattern for the construction of a knitted fabric landing zone, one skilled in the art may benefit from the following exemplary optimization techniques in order to address the requirements of connection performance, structural integrity and frayed edges: (a) For connection performance, a suitable minimum gap [e.g., about 6 mm] should be identified to provide sufficient unbonded areas for optimum male hook engagement. (b) To reduce frayed edges, the standard deviation in both MD and CD directions should be increased [e.g., about 10 %] via an increase in amplitude while also ensuring gap, β, does not become excessively large. Additionally, the minimum % coverage in both MD and CD directions should be increased [e.g., about 31%] (c) For structural integrity, the overall % coverage of the bond pattern should be optimized [e.g., about 40%] and the line width should be optimized [about 2.3 mm], particularly for a lower basis weight knitted fabric. It may be desirable to minimize overall % bonded and the line width of the pattern for connection performance whilst ensuring a sufficient high structural integrity of the knitted fabric bond to the underlying substrate.

In another example, engagement of the hook with the knitted fabric may be increased by the use of pre-stretched and/or elastomeric film layers during the lamination. Such a technique may force the interior pattern portions (i.e., areas without glue) to pop-up during contraction of said film after the lamination stage.

## Claims

1. A knitted fabric female fastening portion for a mechanical fastener **characterized by**: a knitted fabric, said knitted fabric having a first surface and a second surface, said first surface being capable of engaging with a male fastening portion, an underlying substrate, said underlying substrate being joined to said second surface of said knitted fabric, and a bonding layer, said bonding layer being located between said knitted fabric and underlying substrate, said bonding layer bonding said knitted fabric and underlying substrate together, said bonding layer having a first plurality of non-intersecting bond lines and a second plurality of non-intersecting bond lines which are combined to form a pattern of intersecting bond lines that define tessellating pattern elements, wherein said pattern of intersecting bond lines has a gap from about 2 mm to about 6 mm.

2. The knitted fabric female fastening portion of claim 1 wherein said pattern of intersecting bond lines has a CD percent coverage standard deviation not equal to 0% and an MD percent coverage standard deviation not equal to 0%.

3. The knitted fabric female fastening portion of claim 1 wherein said pattern of intersecting bond lines has an amplitude greater than 0 mm.

4. The knitted fabric female fastening portion of claim 3 wherein said amplitude being greater than 0 mm to about 3 mm.

5. The knitted fabric female fastening portion of claim 1 wherein said bond lines are continuous.

6. The knitted fabric female fastening portion of claim 1 wherein said bond lines are discontinuous.

7. The knitted fabric female fastening portion of claim 1 wherein said bond lines are linear.

8. The knitted fabric female fastening portion of claim 1 wherein said bonding layer is an adhesive.

9. A product containing the knitted fabric female fastening portion of claim 1, wherein said product is selected from the group consisting of disposable absorbent articles, body wraps, clothing, packaging, feminine hygiene products, bandages, bibs, food wraps, abrasive systems, cleaning systems and polishing systems.

## Patentansprüche

1. Nach innen gerichteter Maschenstoff-Befestigungsabschnitt für ein mechanisches Befestigungselement, **gekennzeichnet, durch**: einen Maschenstoff, wobei der Maschenstoff eine erste Oberfläche und eine zweite Oberfläche aufweist, wobei die erste Oberfläche mit einem nach außen gerichteten Befestigungsabschnitt in Eingriff gebracht werden kann, ein darunter liegendes Substrat, wobei das darunter liegende Substrat mit der zweiten Oberfläche des Maschenstoffes verbunden ist, und eine Bindeschicht, wobei die Bindeschicht zwischen dem Maschenstoff und dem darunter liegenden Substrat angeordnet ist, wobei die Bindeschicht den Maschenstoff und das darunter liegende Substrat aneinander bindet, wobei die Bindeschicht eine Vielzahl von ersten, sich nicht schneidenden Bindelinien und eine Vielzahl von zweiten, sich nicht schneidenden Bindelinien aufweist, die kombiniert werden, um ein mosaikartiges Muster von sich schneidenden Bindelinien zu bilden, die mosaikartige Musterelemente definieren, wobei das Muster von sich schneidenden Bindelinien einen Spalt von etwa 2 mm bis etwa 6 mm aufweist.

2. Nach innen gerichteter Maschenstoff-Befestigungsabschnitt nach Anspruch 1, wobei das Muster von sich schneidenden Bindelinien eine prozentuale Abdeckungsstandardabweichung in Maschinenquerrichtung von ungleich 0 % und eine prozentuale Abdeckungsstandardabweichung in Maschinenlaufrichtung von ungleich 0 % aufweist.

3. Nach innen gerichteter Maschenstoff-Befestigungsabschnitt nach Anspruch 1, wobei das Muster von sich schneidenden Bindelinien eine Weite von mehr als 0 mm aufweist.

4. Nach innen gerichteter Maschenstoff-Befestigungsabschnitt nach Anspruch 3, wobei die Weite größer als 0 mm bis etwa 3 mm ist.

5. Nach innen gerichteter Maschenstoff-Befestigungsabschnitt nach Anspruch 1, wobei die Bindelinien kontinuierlich sind.

6. Nach innen gerichteter Maschenstoff-Befestigungsabschnitt nach Anspruch 1, wobei die Bindelinien diskontinuierlich sind.

7. Nach innen gerichteter Maschenstoff-Befestigungsabschnitt nach Anspruch 1, wobei die Bindelinien linear sind.

8. Nach innen gerichteter Maschenstoff-Befestigungsabschnitt nach Anspruch 1, wobei die Bindeschicht ein Klebstoff ist.

9. Produkt, enthaltend den nach innen gerichteten Maschenstoff-Befestigungsabschnitt nach Anspruch 1, wobei das Produkt ausgewählt ist aus der Gruppe bestehend aus Einwegabsorptionsartikeln, Körperwickeln, Kleidung, Verpackungen, Damenhygieneprodukten, Bandagen, Lätzchen, Lebensmittelverpackungen, Schleifmittelsystemen, Reinigungssystemen und Poliersystemen.

## Revendications

1. Partie de fixation femelle de tricot pour une fixation mécanique, **caractérisée par** : un tricot, ledit tricot ayant une première surface et une seconde surface, ladite première surface pouvant venir en prise avec une partie de fixation mâle, un substrat sous-jacent, ledit substrat sous-jacent étant relié à ladite seconde surface dudit tricot, et une couche de liaison, ladite couche de liaison étant située entre ledit tricot et ledit substrat sous-jacent, ladite couche de liaison liant ledit tricot et ledit substrat sous-jacent ensemble, ladite couche de liaison ayant une première pluralité de lignes de liaison qui ne se croisent pas et une seconde pluralité de lignes de liaison qui ne se croisent pas qui sont combinées pour former un motif de lignes de liaison qui se croisent qui définissent des éléments de motif en mosaïque, dans laquelle ledit motif de lignes de liaison qui se croisent présente un intervalle d'environ 2 mm à environ 6 mm.

2. Partie femelle de fixation de tricot selon la revendication 1, dans laquelle ledit motif de lignes de liaison qui se croisent a pourcentage d'écart type de couverture CD différent de 0 % et un pourcentage d'écart type de couverture MD différent de 0 %.

3. Partie femelle de fixation de tricot selon la revendication 1, dans laquelle ledit motif de lignes de liaison qui se croisent a une amplitude supérieure à 0 mm.

4. Partie femelle de fixation de tricot selon la revendication 3, dans laquelle ladite amplitude est supérieure à 0 mm jusqu'à environ 3 mm.

5. Partie femelle de fixation de tricot selon la revendication 1, dans laquelle lesdites lignes de liaison sont continues.

6. Partie femelle de fixation de tricot selon la revendication 1, dans laquelle lesdites lignes de liaison sont discontinues.

7. Partie femelle de fixation de tricot selon la revendication 1, dans laquelle lesdites lignes de liaison sont linéaires.

8. Partie femelle de fixation de tricot selon la revendication 1, dans laquelle ladite couche de liaison est un adhésif.

9. Produit contenant la partie de fixation femelle de tricot selon la revendication 1, ledit produit étant choisi dans le groupe constitué par les articles absorbants jetables, les enveloppements corporels, les vêtements, les emballages, les produits d'hygiène féminine, les bandages, les bavoirs, les papiers d'emballage alimentaires, les systèmes abrasifs, les systèmes de nettoyage et les systèmes de polissage.
